# EUROPEAN PATENT SPECIFICATION

(11) **EP 4 353 306 B1**
(45) Date of publication and mention of the grant of the patent: **31.12.2025**
(21) Application number: 22201061.3
(22) Date of filing: 12.10.2022
(51) Int. Cl.: A61N 1/39, A61B 5/361, A61B 5/363, A61B 5/00

(54) **DEFIBRILLATOR FOR ASSESSING A LIKELIHOOD OF PATIENT REFIBRILLATION**
DEFIBRILLATOR ZUR BEURTEILUNG DER WAHRSCHEINLICHKEIT EINER REFIBRILLATION EINES PATIENTEN
DÉFIBRILLATEUR POUR ÉVALUER UNE PROBABILITÉ DE REFIBRILLATION DE PATIENT

(43) Date of publication of application: 17.04.2024
(73) Proprietor: Stryker European Operations Limited, Carrigtwohill, Co. Cork T45 HX08 (IE)
(72) Inventor: DONAGHY, Dymphna, Belfast, BT3 9ED (GB); ANDERSON, Johnny, Belfast, BT3 9ED (GB); HARVEY, Adam, Belfast, BT3 9ED (GB); KERNAGHAN, Amy, Belfast, BT3 9ED (GB); MCALISTER, Olibhear, Belfast, BT3 9ED (GB)
(74) Representative: HGF

(56) References cited:
- US-A1- 2003 191 403
- US-A1- 2013 218 057
- US-A1- 2017 361 120
- US-A1- 2022 233 874

## Description

The invention relates to a defibrillator, particularly but not exclusively, an automated external defibrillator (AED), for assessing a likelihood of refibrillation of a patient and using this to choose a treatment for the patient.

Current AEDs deliver defibrillation shock therapy to patients presenting with a shockable arrythmia, ventricular fibrillation (VF), which is measured using electrocardiogram (ECG) signals of the patient. The defibrillation shock therapy is designed to depolarise the entirety of the myocardium of the patient's heart, remove the arrythmia and restore the coordinated electrical activity of the heart to a normal, or sinus, rhythm. The vast majority of defibrillation shock therapy is successful at terminating VF. However, refibrillation of a patient's heart can occur, where the VF arrythmia returns.

In addition to the delivery of defibrillation shock therapy to the patient, a defibrillator often instructs CPR to be administered to a patient by a user of the defibrillator. CPR compensates for the inability of the patient's heart to pump blood around the body whilst experiencing a VF arrythmia. The time at which CPR is administered has been shown to be important in the treatment of the patient and attaining and maintaining a normal sinus heart rhythm after defibrillation shock therapy.

US 2003/0191403 discloses an implantable medical device and method for assessing autonomic tone and risk factors associated with arrhythmias and, based on this assessment, an early recurrence of ventricular tachycardia or ventricular fibrillation is predicted. Specifically, changes in R-R interval, heart rate variability, patient activity, and myocardial ischemia are measured prior to and after a detected an arrhythmia episode. A recurrence score is calculated as a weighted sum of measured parameters and compared to a prediction criterion. The prediction criterion may be a preset threshold score or an individualized episode template based on previously calculated recurrence scores associated with recurring episodes. Stored parameters and episode-related data may be downloaded for offline analyses for optimizing prediction criteria and monitoring patient status.

US 2013/0218057 discloses an automated external defibrillator (AED) having a treatment decision processor which follows a "shock first" or a "CPR first" rescue protocol after identification of a treatable arrhythmia, depending upon an estimate of the probability of successful resuscitation made from an analysis of a patient parameter measured at the beginning of the rescue. Different CPR protocols may also be followed depending on the estimate. The trend of the measured patient parameter may also be used to adjust the CPR protocol either during a CPR pause or after the initial CPR pause. The AED thus enables an improved rescue protocol.

US 2017/0361120 discloses a defibrillator and method for using a defibrillator which adopts an ECG analysis algorithm that can detect a cardiac arrhythmia in the presence of noise artifact induced by cardio pulmonary resuscitation (CPR) compressions. The apparatus and method includes a confidence analyzer circuit which determines the confidence level of an electrotherapy shock decision based on the detection. If the confidence level is low, the apparatus adjusts its shock decision criteria.

US 2022/233874 discloses an example method performed by a current defibrillator which includes determining that a memory embedded within a therapy cable coupled to the current defibrillator stores data indicative of a previous shock delivered to a patient, the previous shock being delivered using a previous defibrillator. The method also includes obtaining the data indicative of the previous shock, and setting an energy level for a subsequent shock based on the data indicative of the previous shock. The method further includes delivering the subsequent shock to the patient at the energy level for the previous shock.

The invention is set out in the accompanying set of claims.

According to the present invention there is provided a defibrillator for assessing a likelihood of patient refibrillation as defined in claim 1. Preferred features and embodiments of the present invention are the subject of the dependent claims.

The ECG analysis system may analyse the patient ECG signals before a defibrillation shock and generate a ECG pre-shock refibrillation indicator. The defibrillator may use the ECG pre-shock refibrillation indicator to choose treatment for the patient. The defibrillator may use the ECG pre-shock refibrillation indicator to choose to deliver defibrillation shock treatment to the patient at an energy equal to a default energy or deliver defibrillation shock treatment to the patient at an energy greater than the default energy.

The ECG analysis system may analyse the patient ECG signals after delivery of a defibrillation shock and generate at least one ECG post-shock refibrillation indicator. The defibrillator may combine the ECG pre-shock refibrillation indicator and the at least one ECG post-shock refibrillation indicator to generate an aggregate ECG refibrillation indicator. The defibrillator may use the aggregate ECG refibrillation indicator to choose treatment for the patient. The defibrillator may use the aggregate ECG refibrillation indicator to choose to start CPR treatment of the patient or delay CPR treatment of the patient. The defibrillator may issue a start CPR signal or issue a delay CPR signal. The defibrillator may comprise a user interface configured to receive the start CPR signal and deliver a start CPR instruction to a user and to receive the delay CPR signal and deliver a delay CPR instruction to the user.

For a patient experiencing a VF arrythmia, after receiving a defibrillation shock, an ECG signal from the patient may have a waveform exhibiting anything from a continuation of the VF arrythmia, through an irregular partial sinus rhythm, to a regular sinus rhythm. When the ECG signal has a waveform comprising an irregular partial sinus rhythm, the patient's heart will not achieve proper perfusion of blood around the circulatory system. Even when the ECG signal has a waveform comprising a regular sinus rhythm, the patient's heart may not achieve proper perfusion. In these circumstances, CPR is often instructed, to compensate for lack of perfusion. The timing of administration of CPR to a patient influences the likelihood of refibrillation of the patient's heart. Different patients will benefit from shorter or longer delay periods before commencement of CPR. The CPR delay period can be tailored for a patient, based on the expectation of refibrillation of that patient, to reduce the likelihood of refibrillation and the requirement for further defibrillation shock therapy.

Before delivery of a defibrillation shock, the ECG analysis system may take one or more samples of the ECG signals over one or more periods of time. The or each period of time may be in the range of 1 sec to 10 sec. When two or more samples are taken, the periods of time may be any of adjacent periods of time, spaced periods of time.

The ECG analysis system may analyse one or more samples of the ECG signals to detect one or more parameters of the or each sample and may use the or each or at least some of the parameters as one or more inputs of a refibrillation indicator model which generates an ECG pre-shock refibrillation indicator.

The ECG analysis system may analyse one or more whole samples of the ECG signals to detect one or more parameters of the or each whole sample. The ECG analysis system may use the or each or at least some of the parameters as one or more inputs of a refibrillation indicator model which generates an ECG pre-shock refibrillation indicator. The ECG analysis system may aggregate one or more equivalent parameters of multiple whole samples. Aggregation of equivalent parameters may comprise averaging the equivalent parameters. The ECG analysis system may use the or each or at least some of the aggregate parameters as one or more inputs of a refibrillation indicator model which generates an ECG pre-shock refibrillation indicator.

The ECG analysis system may analyse one or more samples of the ECG signals to detect one or more parameters at one or more sample instances of the or each sample. The ECG analysis system may use the or each or at least some of the parameters as one or more inputs of a refibrillation indicator model which generates an ECG pre-shock refibrillation indicator. The ECG analysis system may aggregate one or more equivalent parameters of the one or more samples. Aggregation of equivalent parameters may comprise averaging the equivalent parameters. The ECG analysis system may use the or each or at least some of the aggregate parameters as one or more inputs of a refibrillation indicator model which generates an ECG pre-shock refibrillation indicator.

The one or more parameters may be any of time domain parameters, frequency domain parameters, wavelet parameters. The one or more parameters may be any of an ECG signal sample maximum amplitude, an ECG signal sample minimum amplitude, an ECG signal sample maximum positive slope, an ECG signal sample minimum positive slope, an ECG signal sample maximum negative slope, an ECG signal sample minimum negative slope, an ECG signal sample maximum amplitude spectrum area (AMSA), an ECG signal sample minimum AMSA, an ECG signal sample centroid frequency, an ECG signal sample power. Before delivery of a defibrillation shock, the ECG analysis system may receive ECG signals having a VF waveform, i.e. exhibiting a VF arrythmia. The one or more parameters may be parameters of a VF waveform.

Each of the parameters will correlate with a prospect of success of defibrillation treatment and a likelihood of refibrillation of the patient.

The refibrillation indicator model may weight the or each input according to a strength of indication of refibrillation. The strengths of indication of refibrillation of the inputs may be determined from research. The refibrillation indicator model may use the or each weighted input to generate an ECG pre-shock refibrillation indicator.

For multiple samples, the refibrillation indicator model may generate an ECG pre-shock refibrillation indicator for each sample. For multiple samples, the refibrillation indicator model may aggregate the ECG pre-shock refibrillation indicators to generate an ECG pre-shock refibrillation indicator. For multiple samples, the refibrillation indicator model may weight the ECG pre-shock refibrillation indicators and then aggregate the weighted ECG pre-shock refibrillation indicators to generate an ECG pre-shock refibrillation indicator.

The refibrillation indicator model may compare the ECG pre-shock refibrillation indicator to at least one pre-determined ECG pre-shock refibrillation threshold and generate a flag which prompts the defibrillator to deliver a treatment to the patient. The at least one pre-determined ECG pre-shock refibrillation threshold may be generated using data from patients who have received defibrillation shock therapy. The at least one pre-determined ECG pre-shock refibrillation threshold may be generated using data from the patient receiving defibrillation shock therapy. When the ECG pre-shock refibrillation indicator is less than or equal to the pre-determined ECG pre-shock refibrillation threshold, the refibrillation indicator model may generate a flag which prompts the defibrillator to deliver defibrillation shock treatment to the patient at an energy equal to a default energy. When the ECG pre-shock refibrillation indicator is greater than the pre-determined ECG pre-shock refibrillation threshold, the refibrillation indicator model may generate a flag which prompts the defibrillator to deliver defibrillation shock treatment to the patient at an energy greater than the default energy.

The refibrillation indicator model may be a binary logistic regression model. The regression model may determine a likelihood of refibrillation using the one or more inputs of the model.

After delivery of a defibrillation shock, the ECG analysis system may take one or more samples of the ECG signals over one or more periods of time. The or each period of time may be in the range of 1 sec to 10 sec. When two or more samples are taken, the periods of time may be any of adjacent periods of time, spaced periods of time. The period of time may comprise a moving window of time.

The ECG analysis system may analyse one or more samples of the ECG signals to detect one or more parameters of the or each sample and may use the or each or at least some of the parameters as one or more inputs of a refibrillation indicator model which generates an ECG post-shock refibrillation indicator.

The ECG analysis system may analyse one or more whole samples of the ECG signals to detect one or more parameters of the or each whole sample. The ECG analysis system may use the or each or at least some of the parameters as one or more inputs of a refibrillation indicator model which generates an ECG post-shock refibrillation indicator. The ECG analysis system may aggregate one or more equivalent parameters of multiple whole samples. Aggregation of equivalent parameters may comprise averaging the equivalent parameters. The ECG analysis system may use the or each or at least some of the aggregate parameters as one or more inputs of a refibrillation indicator model which generates an ECG post-shock refibrillation indicator.

The ECG analysis system may analyse one or more samples of the ECG signals to detect one or more parameters at one or more sample instances of the or each sample. The ECG analysis system may use the or each or at least some of the parameters as one or more inputs of a refibrillation indicator model which generates an ECG post-shock refibrillation indicator. The ECG analysis system may aggregate one or more equivalent parameters of the one or more samples. Aggregation of equivalent parameters may comprise averaging the equivalent parameters. The ECG analysis system may use the or each or at least some of the aggregate parameters as one or more inputs of a refibrillation indicator model which generates an ECG post-shock refibrillation indicator.

The one or more parameters may be any of time domain parameters, frequency domain parameters, wavelet parameters. The one or more parameters may be any of an ECG signal sample maximum amplitude, an ECG signal sample minimum amplitude, an ECG signal sample maximum positive slope, an ECG signal sample minimum positive slope, an ECG signal sample maximum negative slope, an ECG signal sample minimum negative slope, an ECG signal sample maximum AMSA, an ECG signal sample minimum AMSA, an ECG signal sample centroid frequency, an ECG signal sample power.

After delivery of a defibrillation shock, the ECG analysis system may receive ECG signals having a waveform exhibiting one or more PQRST complexes. The one or more parameters may comprise any of an amplitude of a P wave of a PQRST complex, an amplitude of a QRS wave of a PQRST complex, variation of amplitudes of QRS waves of two or more PQRST complexes, an amplitude of a T wave of a PQRST complex, a width of a P wave of a PQRST complex, a width of a QRS wave of a PQRST complex, a width of a T wave of a PQRST complex, a ST segment of a PQRST complex, a coupling interval between a QRS complex and a premature, abnormal complex, an R-R interval between two or more PQRST complexes, a ST deviation, presence of one or more premature ventricular contractions.

Each of the parameters will correlate with a prospect of success of defibrillation treatment and a likelihood of refibrillation of the patient.

The refibrillation indicator model may weight the or each input according to a strength of indication of refibrillation. The strengths of indication of refibrillation of the inputs may be determined from research. The refibrillation indicator model may use the or each weighted input to generate an ECG post-shock refibrillation indicator.

For multiple samples, the refibrillation indicator model may generate an ECG post-shock refibrillation indicator for each sample. For multiple samples, the refibrillation indicator model may aggregate the ECG post-shock refibrillation indicators to generate an ECG post-shock refibrillation indicator. For multiple samples, the refibrillation indicator model may weight the ECG post-shock refibrillation indicators and then aggregate the weighted ECG post-shock refibrillation indicators to generate an ECG post-shock refibrillation indicator.

The refibrillation indicator model may be a binary logistic regression model. The regression model may determine a likelihood of refibrillation using the one or more inputs of the model.

The refibrillation indicator model may combine the ECG pre-shock refibrillation indicator and the ECG post-shock refibrillation indicator to generate an aggregate ECG refibrillation indicator. The refibrillation indicator model may combine the ECG pre-shock refibrillation indicator and the ECG post-shock refibrillation indicator by any of summing the ECG pre-shock refibrillation indicator and the ECG post-shock refibrillation indicator, taking a product of the ECG pre-shock refibrillation indicator and the ECG post-shock refibrillation indicator, taking a ratio of the ECG pre-shock refibrillation indicator to the ECG post-shock refibrillation indicator, using a model to combine the ECG pre-shock refibrillation indicator and the ECG post-shock refibrillation indicator.

The refibrillation indicator model may weight the ECG pre-shock refibrillation indicator and may weight the ECG post-shock refibrillation indicator. The refibrillation indicator model may combine the weighted ECG pre-shock refibrillation indicator and the weighted ECG post-shock refibrillation indicator to generate an aggregate ECG refibrillation indicator. The refibrillation indicator model may combine the weighted ECG pre-shock refibrillation indicator and the weighted ECG post-shock refibrillation indicator by any of summing the weighted ECG pre-shock refibrillation indicator and the weighted ECG post-shock refibrillation indicator, taking a product of the weighted ECG pre-shock refibrillation indicator and the weighted ECG post-shock refibrillation indicator, taking a ratio of the weighted ECG pre-shock refibrillation indicator to the weighted ECG post-shock refibrillation indicator, using a model to combine the weighted ECG pre-shock refibrillation indicator and the weighted ECG post-shock refibrillation indicator.

The refibrillation indicator model may compare the aggregate ECG refibrillation indicator with at least one pre-determined aggregate ECG refibrillation threshold and generate a flag which prompts the defibrillator to deliver a treatment to the patient. The at least one pre-determined aggregate ECG refibrillation threshold may be generated using data from patients who have received defibrillation shock therapy. The at least one pre-determined aggregate ECG refibrillation threshold may be generated using data from the patient receiving defibrillation shock therapy. When the aggregate ECG refibrillation indicator is less than or equal to the at least one pre-determined ECG refibrillation threshold, the refibrillation indicator model may generate a flag which prompts the defibrillator to start CPR treatment of the patient. The defibrillator may issue a start CPR signal. The user interface of the defibrillator may receive the start CPR signal and deliver a start CPR instruction to a user. When the aggregate ECG refibrillation indicator is greater than the at least one pre-determined ECG refibrillation threshold, the refibrillation indicator model may generate a flag which prompts the defibrillator to delay CPR treatment of the patient. The defibrillator may issue a delay CPR signal. The user interface of the defibrillator may receive the delay CPR signal and deliver a delay CPR instruction to the user.

When the aggregate or weighted aggregate ECG refibrillation indicator, which represents a likelihood of refibrillation of the patient, is low, the patient has a lower likelihood of refibrillation and immediate CPR can be commenced. When the aggregate or weighted aggregate ECG refibrillation indicator is high, the patient has a higher likelihood of refibrillation and delayed CPR is recommended.

At one or more further times after delivery of the defibrillation shock, the ECG analysis system may repeat analysis of the patient ECG signal and the refibrillation indicator model may generate a further ECG post-shock refibrillation indicator. The refibrillation indicator model may combine the ECG pre-shock refibrillation indicator and the further ECG post-shock refibrillation indicator to generate an aggregate ECG refibrillation indicator.

The refibrillation indicator model may compare the aggregate ECG refibrillation indicator with at least one pre-determined aggregate ECG refibrillation threshold and generate a flag which prompts the defibrillator to deliver a treatment to the patient. The at least one pre-determined aggregate ECG refibrillation threshold may be generated using data from patients who have received defibrillation shock therapy. The at least one pre-determined aggregate ECG refibrillation threshold may be generated using data from the patient receiving defibrillation shock therapy. When the aggregate ECG refibrillation indicator is less than or equal to the at least one pre-determined ECG refibrillation threshold, the refibrillation indicator model may generate a flag which prompts the defibrillator to start CPR treatment of the patient. The defibrillator may issue a start CPR signal. The user interface of the defibrillator may receive the start CPR signal and deliver a start CPR instruction to a user. When the aggregate ECG refibrillation indicator is greater than the at least one pre-determined ECG refibrillation threshold, the refibrillation indicator model may generate a flag which prompts the defibrillator to delay CPR treatment of the patient. The defibrillator may issue a delay CPR signal. The user interface of the defibrillator may receive the delay CPR signal and deliver a delay CPR instruction to the user.

The ECG analysis system may analyse the one or more samples of the ECG signal by operating one or more ECG signal analysis algorithms. The ECG analysis system may operate the one or more ECG signal analysis algorithms by executing software instructions of the or each algorithm. The ECG analysis system may comprise at least one ECG analysis processor configured to operate the one or more ECG signal analysis algorithms by executing software instructions of the or each algorithm.

The defibrillator further comprises an impedance signal input configured to receive impedance signals of the patient and an impedance analysis system, in communication with the impedance signal input, configured to receive the patient impedance signal, analyse the patient impedance signal to generate at least one impedance refibrillation indicator representing a likelihood of refibrillation of the patient. The defibrillator may use the at least one impedance refibrillation indicator to choose treatment for the patient. The defibrillator may use the impedance refibrillation indicator to choose to start CPR treatment of the patient or delay CPR treatment of the patient. The defibrillator may issue a start CPR signal or issue a delay CPR signal. The defibrillator may comprise a user interface configured to receive the start CPR signal and deliver a start CPR instruction to a user and to receive the delay CPR signal and deliver a delay CPR instruction to the user.

The refibrillation indicator model may combine the aggregate ECG refibrillation indicator and the at least one impedance refibrillation indicator to generate an aggregate ECG/impedance refibrillation indicator. The refibrillation indicator model may compare the aggregate ECG/impedance refibrillation indicator with an aggregate ECG/impedance refibrillation threshold and generate a flag which prompts the defibrillator to deliver a treatment to the patient. When the aggregate ECG/impedance refibrillation indicator is less than or equal to the aggregate ECG/impedance refibrillation threshold, the refibrillation indicator model may generate a flag which prompts the defibrillator to commence CPR. The defibrillator may issue a commence CPR signal. When the aggregate ECG/impedance refibrillation indicator is greater than the aggregate ECG/impedance refibrillation threshold, the refibrillation indicator model may generate a flag which prompts the defibrillator to delay CPR. The defibrillator may issue a delay CPR signal.

After delivery of a defibrillation shock, the impedance analysis system may analyse the patient impedance signal to detect one or more impedance waveforms indicating one or more contractions of the patient's heart. The impedance analysis system may analyse the patient impedance signals to detect the one or more impedance waveforms indicating one or more contractions of the patient's heart by searching for the one or more impedance waveforms at positions corresponding to positions of PQRST complexes in the patient ECG signal. The impedance analysis system may process the one or more impedance waveforms to measure one or more parameters of the one or more impedance waveforms. The one or more parameters of the one or more impedance waveforms may comprise an amplitude of a peak of an impedance waveform, a width of a peak of an impedance waveform. The impedance analysis system may use the one or more parameters of the one or more impedance waveforms to generate a first impedance post-shock refibrillation indicator.

After delivery of a defibrillation shock, the impedance analysis system may analyse the patient impedance signal to detect one or more impedance waveforms indicating one or more breaths of the patient. The impedance analysis system may analyse the patient impedance signals to detect the one or more impedance waveforms indicating one or more breaths of the patient by using an artificial intelligence or a machine learning technique, which teaches the defibrillator to identify impedance waveforms that indicate one or more patient breaths. The impedance analysis system may process the one or more impedance waveforms to measure one or more parameters of the one or more impedance waveforms. The impedance analysis system may use the one or more parameters of the one or more impedance waveforms to generate a second impedance post-shock refibrillation indicator.

The refibrillation indicator model may combine the aggregate ECG refibrillation indicator and the first impedance post-shock refibrillation indicator and/or the second impedance post-shock refibrillation indicator to generate an aggregate ECG/impedance refibrillation indicator. The refibrillation indicator model may compare the aggregate ECG/impedance refibrillation indicator with an aggregate ECG/impedance refibrillation threshold and generate a flag which prompts the defibrillator to deliver a treatment to the patient. When the aggregate ECG/impedance refibrillation indicator is less than or equal to the aggregate ECG/impedance refibrillation threshold, the refibrillation indicator model may generate a flag which prompts the defibrillator to commence CPR. The defibrillator may issue a commence CPR signal. When the aggregate ECG/impedance refibrillation indicator is greater than the aggregate ECG/impedance refibrillation threshold, the refibrillation indicator model may generate a flag which prompts the defibrillator to delay CPR. The defibrillator may issue a delay CPR signal.

The refibrillation indicator model may weight the aggregate ECG refibrillation indicator and the first and second impedance refibrillation indicators. The refibrillation indicator model may combine the weighted aggregate ECG refibrillation indicator and the weighted first impedance post-shock refibrillation indicator and/or the weighted second impedance post-shock refibrillation indicator to generate an aggregate ECG/impedance refibrillation indicator.

The refibrillation indicator model may compare the aggregate ECG/impedance refibrillation indicator with an aggregate ECG/impedance refibrillation threshold and generate a flag which prompts the defibrillator to deliver a treatment to the patient. When the aggregate ECG/impedance refibrillation indicator is less than or equal to the aggregate ECG/impedance refibrillation threshold, the refibrillation indicator model may generate a flag which prompts the defibrillator to commence CPR. The defibrillator may issue a commence CPR signal. When the aggregate ECG/impedance refibrillation indicator is greater than the aggregate ECG/impedance refibrillation threshold, the refibrillation indicator model may generate a flag which prompts the defibrillator to delay CPR. The defibrillator may issue a delay CPR signal.

The aggregate ECG/impedance refibrillation threshold may be a predetermined threshold generated using data from patients who have received defibrillation shock therapy. The aggregate ECG/impedance refibrillation threshold may be a patient-based threshold generated using treatment history data of the patient.

The user interface may comprise an audio communication unit, such as a speaker, configured to receive the start CPR signal and deliver an audible start CPR instruction to the user and to receive the delay CPR signal and deliver an audible delay CPR instruction to the user. Additionally or alternatively, the user interface may comprise a visual communication unit, such as a display, configured to receive the start CPR signal and deliver a visual start CPR instruction to the user and to receive the delay CPR signal and deliver a visual delay CPR instruction to the user.

The defibrillator may comprise a defibrillation shock circuit configured to generate and deliver one or more defibrillation shocks to the patient.

The defibrillator may comprise a plurality of ECG electrodes adapted for attachment to the patient. The ECG electrodes may measure an ECG signal of the patient. The ECG electrodes may be in communication with the ECG signal input for the input to receive the ECG signal of the patient.

The defibrillator may comprise a plurality of impedance electrodes adapted for attachment to the patient. The impedance electrodes may measure an impedance signal of the patient. The impedance electrodes may be in communication with the impedance signal input for the input to receive the impedance signal of the patient.

The invention will now be described, by way of example only, with reference to the accompanying drawings in which:
Figure 1 is a schematic representation of a defibrillator according to the invention, and
Figure 2 is a flow chart of an example process carried out by the defibrillator of Figure 1.

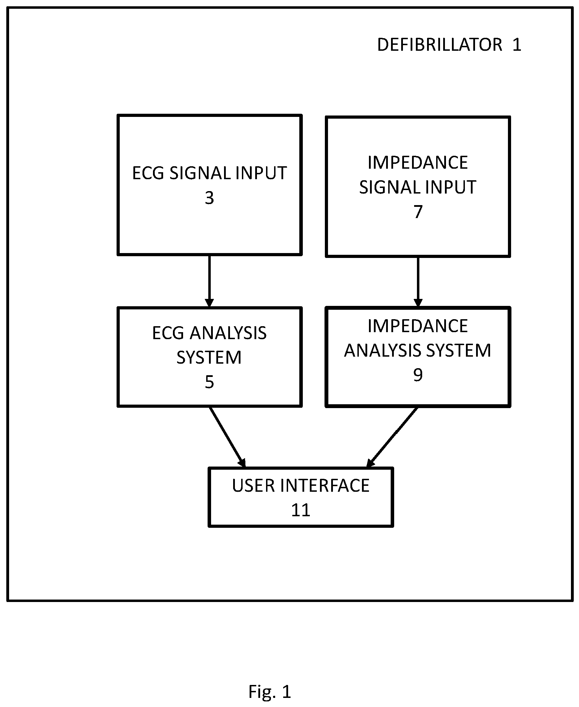

Referring to Figure 1, a defibrillator 1 for assessing patient refibrillation, comprises an ECG signal input 3, configured to receive ECG signals of the patient, an ECG analysis system 5, in communication with the ECG signal input 3, an impedance signal input 7, configured to receive impedance signals of the patient, an impedance analysis system 9, in communication with the impedance signal input 7 and a user interface 11.

The defibrillator 1 comprises a plurality of ECG electrodes (not shown) adapted for attachment to the patient. The ECG electrodes measure an ECG signal of the patient and are in communication with the ECG signal input 3 for the input to receive the ECG signal of the patient. The defibrillator 1 further comprises a plurality of impedance electrodes (not shown) adapted for attachment to the patient. The impedance electrodes measure an impedance signal of the patient and are in communication with the impedance signal input 7 for the input to receive the impedance signal of the patient.

The ECG analysis system 5 and the impedance analysis system 9 comprise one or more processors (not shown), or other suitable hardware, to run one or more software algorithms for the analysis of the patient ECG signals and patient impedance signals.

The defibrillator 1 comprises a defibrillation shock circuit (not shown) configured to generate and deliver one or more defibrillation shocks to the patient.

It will be appreciated that the defibrillator 1 comprises other components, such as a power supply, which are not illustrated in Figure 1.

Generally, the defibrillator 1 operates to receive the patient ECG signals, analyse the patient ECG signals to generate an ECG refibrillation indicator representing a likelihood of refibrillation of the patient and use the ECG refibrillation indicator to choose treatment for the patient. The defibrillator further operates to receive the patient impedance signals, analyse the patient impedance signals to generate an impedance refibrillation indicator representing a likelihood of refibrillation of the patient and use the impedance refibrillation indicator to choose treatment for the patient.

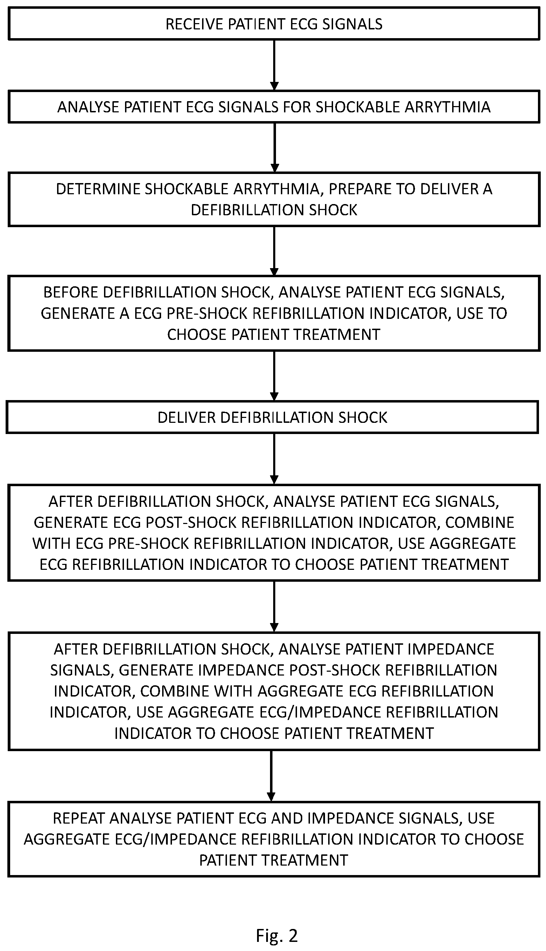

Referring to Figure 2, an example process carried out by the defibrillator 1 will be described. After application of the ECG electrodes to the patient, the defibrillator 1 the ECG signal input 3 receives patient ECG signals from the ECG electrodes. The defibrillator uses at least one algorithm to analyse the received ECG signals for a shockable arrythmia. The algorithm may be a set of software instructions run by the ECG analysis system 5 or another analysis component of the defibrillator 1. When a shockable arrythmia is determined, such as VF, the defibrillator 1 begins preparations to deliver a defibrillation shock.

The ECG analysis system 5 analyses the patient ECG signals before a defibrillation shock, generates a ECG pre-shock refibrillation indicator and uses this to choose treatment for the patient. Specifically, in one embodiment, the ECG analysis system 5 uses the ECG pre-shock refibrillation indicator to choose to deliver defibrillation shock treatment to the patient at an energy equal to a default energy or deliver defibrillation shock treatment to the patient at an energy greater than the default energy.

The ECG analysis system 5 also analyses the patient ECG signals after a defibrillation shock and generates at least one ECG post-shock refibrillation indicator. This is combined with the ECG pre-shock refibrillation indicator to generate an aggregate ECG refibrillation indicator, which is used to choose treatment for the patient. Specifically, in one embodiment, the ECG analysis system 5 uses the aggregate ECG refibrillation indicator to choose to start CPR treatment of the patient or delay CPR treatment of the patient.

In this embodiment, the impedance analysis system 9 analyses the patient impedance signals after delivery of a defibrillation shock and generates at least one impedance refibrillation indicator. This is combined with the aggregate ECG refibrillation indicator to generate an aggregate ECG/impedance refibrillation indicator, which is used to choose treatment for the patient. Specifically, in one embodiment, the impedance analysis system 9 uses the aggregate ECG/impedance refibrillation indicator to choose to start CPR treatment of the patient or delay CPR treatment of the patient.

The ECG analysis system 5 and the impedance analysis system 9 issue a start CPR signal or issue a delay CPR signal, which is received by the user interface 11 which delivers a start CPR instruction to a user of the defibrillator 1 or a delay CPR instruction to the user. The user interface 11 comprises an audio communication unit, such as a speaker, configured to receive the start CPR signal and deliver an audible start CPR instruction to the user and to receive the delay CPR signal and deliver an audible delay CPR instruction to the user. Additionally or alternatively, the user interface 11 comprises a visual communication unit, such as a display, configured to receive the start CPR signal and deliver a visual start CPR instruction to the user and to receive the delay CPR signal and deliver a visual delay CPR instruction to the user.

Repeated analysis of the patient ECG and impedance signals is carried out and further aggregate ECG/impedance refibrillation indicators are generated and used to choose treatment for the patient.

When patient treatment comprising CPR delay is chosen, repeated analysis of the patient ECG signals is carried out. If during the delay, a shockable patient heart arrythmia is detected, the defibrillator 1 prepares for and delivers a defibrillation shock.

It will be appreciated that, in other embodiments, analysis of patient impedance signals may be omitted and the patient treatment chosen using ECG refibrillation indicators only.

In more detail, before delivery of a defibrillation shock, the ECG analysis system 5 takes one or more samples of the ECG signals over one or more periods of time. The or each period of time may be in the range of 1 sec to 10 sec. When two or more samples are taken, the periods of time may be any of adjacent periods of time, spaced periods of time.

In this embodiment, the ECG analysis system 5 analyses one or more whole samples of the ECG signals to detect one or more parameters of the or each whole sample. The ECG analysis system 5 then uses the or each or at least some of the parameters and/or aggregated parameters as one or more inputs of a refibrillation indicator model which generates an ECG pre-shock refibrillation indicator.

Alternatively, the ECG analysis system 5 may analyse one or more samples of the ECG signals to detect one or more parameters at one or more sample instances of the or each sample and use the or each or at least some of the parameters and/or aggregated parameters as one or more inputs of a refibrillation indicator model which generates an ECG pre-shock refibrillation indicator.

Before delivery of a defibrillation shock, it is expected that the ECG analysis system 5 receives ECG signals having an arrythmia waveform, e.g. a VF waveform. The one or more parameters will then be parameters of the VF waveform.

The one or more parameters may be any of time domain parameters, frequency domain parameters, wavelet parameters. The one or more parameters may be any of an ECG signal sample maximum amplitude, an ECG signal sample minimum amplitude, an ECG signal sample maximum positive slope, an ECG signal sample minimum positive slope, an ECG signal sample maximum negative slope, an ECG signal sample minimum negative slope, an ECG signal sample maximum amplitude spectrum area (AMSA), an ECG signal sample minimum AMSA, an ECG signal sample centroid frequency, an ECG signal sample power.

The refibrillation indicator model may weight each input according to its strength of indication of refibrillation. The strengths of indication of refibrillation of the inputs may be determined from research. The refibrillation indicator model may use the weighted inputs to generate an ECG pre-shock refibrillation indicator.

For multiple samples, the refibrillation indicator model may generate an ECG pre-shock refibrillation indicator for each sample and aggregate the ECG pre-shock refibrillation indicators to generate an ECG pre-shock refibrillation indicator. For multiple samples, the refibrillation indicator model may weight the ECG pre-shock refibrillation indicators and then aggregate the weighted ECG pre-shock refibrillation indicators to generate an ECG pre-shock refibrillation indicator.

The refibrillation indicator model may be a binary logistic regression model. The regression model may determine a likelihood of refibrillation using the one or more inputs of the model.

The refibrillation indicator model compares the ECG pre-shock refibrillation indicator to at least one pre-determined ECG pre-shock refibrillation threshold and generates a flag which prompts the defibrillator to deliver a treatment to the patient. The at least one pre-determined ECG pre-shock refibrillation threshold may be generated using data from patients who have received defibrillation shock therapy and/or using data from the patient receiving defibrillation shock therapy. When the ECG pre-shock refibrillation indicator is less than or equal to the pre-determined ECG pre-shock refibrillation threshold, the refibrillation indicator model generates a flag which prompts the defibrillator 1 to deliver defibrillation shock treatment to the patient at an energy equal to a default energy. When the ECG pre-shock refibrillation indicator is greater than the pre-determined ECG pre-shock refibrillation threshold, the refibrillation indicator model generates a flag which prompts the defibrillator 1 to deliver defibrillation shock treatment to the patient at an energy greater than the default energy.

After delivery of the defibrillation shock, the ECG analysis system 5 take one or more samples of the ECG signals over one or more periods of time. In this embodiment, the ECG analysis system 5 analyse one or more whole samples of the ECG signal to detect one or more parameters of the or each whole sample and uses the or each or at least some of the parameters and/or aggregated parameters as one or more inputs of a refibrillation indicator model which generates an ECG post-shock refibrillation indicator.

In other embodiments, the ECG analysis system 5 may, additionally or alternatively, analyse one or more samples of the ECG signal to detect one or more parameters at one or more sample instances of the or each sample and use the or each or at least some of the parameters and/or aggregated parameters as one or more inputs of a refibrillation indicator model which generates an ECG post-shock refibrillation indicator.

The one or more parameters may be any of time domain parameters, frequency domain parameters, wavelet parameters. The one or more parameters may be any of an ECG signal sample maximum amplitude, an ECG signal sample minimum amplitude, an ECG signal sample maximum positive slope, an ECG signal sample minimum positive slope, an ECG signal sample maximum negative slope, an ECG signal sample minimum negative slope, an ECG signal sample maximum AMSA, an ECG signal sample minimum AMSA, an ECG signal sample centroid frequency, an ECG signal sample power.

After delivery of a defibrillation shock, it is expected that the ECG analysis system 5 receives ECG signals having a waveform exhibiting one or more PQRST complexes. The one or more parameters may comprise any of an amplitude of a P wave of a PQRST complex, an amplitude of a QRS wave of a PQRST complex, variation of amplitudes of QRS waves of two or more PQRST complexes, an amplitude of a T wave of a PQRST complex, a width of a P wave of a PQRST complex, a width of a QRS wave of a PQRST complex, a width of a T wave of a PQRST complex, a ST segment of a PQRST complex, a coupling interval between a QRS complex and a premature, abnormal complex, an R-R interval between two or more PQRST complexes, a ST deviation, presence of one or more premature ventricular contractions.

The refibrillation indicator model may weight each input according to its strength of indication of refibrillation. The strengths of indication of refibrillation of the inputs may be determined from research. The refibrillation indicator model may use the weighted inputs to generate an ECG post-shock refibrillation indicator.

For multiple samples, the refibrillation indicator model may generate an ECG post-shock refibrillation indicator for each sample. For multiple samples, the refibrillation indicator model may aggregate the ECG post-shock refibrillation indicators to generate an ECG post-shock refibrillation indicator. For multiple samples, the refibrillation indicator model may weight the ECG post-shock refibrillation indicators and then aggregate the weighted ECG post-shock refibrillation indicators to generate an ECG post-shock refibrillation indicator.

The refibrillation indicator model may be a logistic regression model. The logistical regression model may determine a likelihood of refibrillation using the one or more inputs of the model.

The refibrillation indicator model combines the ECG pre-shock refibrillation indicator and the ECG post-shock refibrillation indicator to generate an aggregate ECG refibrillation indicator. The refibrillation indicator model may weight the ECG pre-shock refibrillation indicator, weight the ECG post-shock refibrillation indicator and combine the weighted ECG pre-shock refibrillation indicator and the weighted ECG post-shock refibrillation indicator to generate an aggregate ECG refibrillation indicator.

The refibrillation indicator model compares the aggregate ECG refibrillation indicator with at least one pre-determined aggregate ECG refibrillation threshold and generates a flag which prompts the defibrillator to deliver a treatment to the patient. The at least one pre-determined aggregate ECG refibrillation threshold may be generated using data from patients who have received defibrillation shock therapy and/or using data from the patient receiving defibrillation shock therapy. When the aggregate ECG refibrillation indicator is less than or equal to the at least one pre-determined ECG refibrillation threshold, the refibrillation indicator model generates a flag which prompts the defibrillator 1 to start CPR treatment of the patient and to issue a start CPR signal. The user interface 11 of the defibrillator 1 receives the start CPR signal and delivers a start CPR instruction to the user. When the aggregate ECG refibrillation indicator is greater than the at least one pre-determined ECG refibrillation threshold, the refibrillation indicator model generates a flag which prompts the defibrillator 1 to delay CPR treatment of the patient and to issue a delay CPR signal. The user interface 11 of the defibrillator 1 receives the delay CPR signal and delivers a delay CPR instruction to the user.

At one or more further times after delivery of the defibrillation shock, the ECG analysis system repeats analysis of the patient ECG signals and the refibrillation indicator model generates a further ECG post-shock refibrillation indicator. The refibrillation indicator model combines the ECG pre-shock refibrillation indicator and the further ECG post-shock refibrillation indicator to generate an aggregate ECG refibrillation indicator.

The refibrillation indicator model compares the aggregate ECG refibrillation indicator with at least one pre-determined aggregate ECG refibrillation threshold and generates a flag which prompts the defibrillator to deliver a treatment to the patient. When the aggregate ECG refibrillation indicator is less than or equal to the at least one pre-determined ECG refibrillation threshold, the refibrillation indicator model again generates a flag which prompts the defibrillator 1 to start CPR treatment of the patient and to issue a start CPR signal. When the aggregate ECG refibrillation indicator is greater than the at least one pre-determined ECG refibrillation threshold, the refibrillation indicator model generates a flag which prompts the defibrillator 1 to delay CPR treatment of the patient and to issue a delay CPR signal.

In this embodiment, after delivery of the defibrillation shock, the impedance analysis system 9 analyses the patient impedance signals to detect one or more impedance waveforms indicating one or more contractions of the patient's heart, by searching for the one or more impedance waveforms at positions corresponding to positions of PQRST complexes in the patient ECG signals. The impedance analysis system 9 processes the one or more impedance waveforms to measure one or more parameters. The one or more parameters may comprise an amplitude of a peak of an impedance waveform, a width of a peak of an impedance waveform. The impedance analysis system 9 uses the one or more parameters of the one or more impedance waveforms to generate a first impedance post-shock refibrillation indicator.

The impedance analysis system 9 further analyses the patient impedance signal to detect one or more impedance waveforms indicating one or more breaths of the patient by using an artificial intelligence or a machine learning technique, which teaches the defibrillator to identify impedance waveforms that indicate one or more patient breaths. The impedance analysis system 9 processes the one or more impedance waveforms to measure one or more parameters and uses the one or more parameters to generate a second impedance post-shock refibrillation indicator.

The refibrillation indicator model combines the aggregate ECG refibrillation indicator and the first impedance post-shock refibrillation indicator and the second impedance post-shock refibrillation indicator to generate an aggregate ECG/impedance refibrillation indicator. The refibrillation indicator model compares the aggregate ECG/impedance refibrillation indicator to an aggregate ECG/impedance refibrillation threshold.

The refibrillation indicator model may weight the aggregate ECG refibrillation indicator and the first and second impedance refibrillation indicators. The refibrillation indicator model may combine the weighted aggregate ECG refibrillation indicator and the weighted first impedance post-shock refibrillation indicator and the weighted second impedance post-shock refibrillation indicator to generate an aggregate ECG/impedance refibrillation indicator.

The refibrillation indicator model compares the aggregate ECG/impedance refibrillation indicator with an aggregate ECG/impedance refibrillation threshold. When the aggregate ECG/impedance refibrillation indicator is less than or equal to the aggregate ECG/ impedance refibrillation threshold, the refibrillation indicator model prompts the defibrillator 1 to issue a commence CPR signal. When the aggregate ECG/impedance refibrillation indicator is greater than the aggregate ECG/impedance refibrillation threshold, the refibrillation indicator model prompts the defibrillator 1 to issue a delay CPR signal.

During any CPR delay time, the ECG analysis system 5 receives and analyses patient ECG signals. If a shockable arrythmia is determined, the defibrillator 1 delivers a further defibrillation shock.

The present invention is defined by the appended claims.

## Claims

1. A defibrillator (1) for assessing a likelihood of patient refibrillation, comprising:
an ECG signal input (3) configured to receive ECG signals of the patient;
an ECG analysis system (5), in communication with the ECG signal input (3), configured to receive the patient ECG signals, analyse the patient ECG signals to generate an ECG refibrillation indicator representing a likelihood of refibrillation of the patient and use the ECG refibrillation indicator to choose treatment for the patient;
an impedance signal input (7) configured to receive impedance signals of the patient, and
an impedance analysis system (9), in communication with the impedance signal input (7), configured to receive the patient impedance signal,
**characterised in that:**
the impedance analysis system (9) is configured to analyse the patient impedance signal to generate at least one impedance refibrillation indicator representing a likelihood of refibrillation of the patient and the defibrillator is configured to also use the at least one impedance refibrillation indicator to choose treatment for the patient.

2. A defibrillator (1) according to claim 1 in which the ECG analysis system (5) is configured to analyse the patient ECG signals before a defibrillation shock and generates a ECG pre-shock refibrillation indicator and the defibrillator (1) uses the ECG pre-shock refibrillation indicator to choose treatment for the patient.

3. A defibrillator (1) according to claim 2 which is configured to use the ECG pre-shock refibrillation indicator to choose to deliver defibrillation shock treatment to the patient at an energy equal to a default energy or deliver defibrillation shock treatment to the patient at an energy greater than the default energy.

4. A defibrillator (1) according to claim 2 or claim 3 in which the ECG analysis system (5) is configured to analyse the patient ECG signals after delivery of a defibrillation shock and generates at least one ECG post-shock refibrillation indicator and the defibrillator (1) is configured to combine the ECG pre-shock refibrillation indicator and the at least one ECG post-shock refibrillation indicator to generate an aggregate ECG refibrillation indicator and to use the aggregate ECG refibrillation indicator to choose treatment for the patient.

5. A defibrillator (1) according to claim 4 which is configured to use the aggregate ECG refibrillation indicator to choose to start CPR treatment of the patient or delay CPR treatment of the patient.

6. A defibrillator (1) according to any preceding claim in which, before delivery of a defibrillation shock, the ECG analysis system (5) is configured to take one or more samples of the ECG signals over one or more periods of time, to analyse one or more samples of the ECG signals to detect one or more parameters of the or each sample and to use the or each or at least some of the parameters as one or more inputs of a refibrillation indicator model which is configured to generate an ECG pre-shock refibrillation indicator.

7. A defibrillator (1) according to claim 6 in which the one or more parameters are any of time domain parameters, frequency domain parameters, wavelet parameters, an ECG signal sample maximum amplitude, an ECG signal sample minimum amplitude, an ECG signal sample maximum positive slope, an ECG signal sample minimum positive slope, an ECG signal sample maximum negative slope, an ECG signal sample minimum negative slope, an ECG signal sample maximum amplitude spectrum area (AMSA), an ECG signal sample minimum AMSA, an ECG signal sample centroid frequency, an ECG signal sample power, parameters of a VF waveform.

8. A defibrillator (1) according to claim 6 or claim 7 in which the refibrillation indicator model is configured to compare the ECG pre-shock refibrillation indicator to at least one pre-determined ECG pre-shock refibrillation threshold and to generate a flag which prompts the defibrillator (1) to deliver a treatment to the patient.

9. A defibrillator (1) according to any preceding claim in which, after delivery of a defibrillation shock, the ECG analysis system (5) is configured to take one or more samples of the ECG signals over one or more periods of time, to analyse one or more samples of the ECG signals to detect one or more parameters of the or each sample and to use the or each or at least some of the parameters as one or more inputs of a refibrillation indicator model which generates an ECG post-shock refibrillation indicator.

10. A defibrillator (1) according to claim 9 in which the one or more parameters are any of time domain parameters, frequency domain parameters, wavelet parameters, an ECG signal sample maximum amplitude, an ECG signal sample minimum amplitude, an ECG signal sample maximum positive slope, an ECG signal sample minimum positive slope, an ECG signal sample maximum negative slope, an ECG signal sample minimum negative slope, an ECG signal sample maximum AMSA, an ECG signal sample minimum AMSA, an ECG signal sample centroid frequency, an ECG signal sample power, an amplitude of a P wave of a PQRST complex, an amplitude of a QRS wave of a PQRST complex, variation of amplitudes of QRS waves of two or more PQRST complexes, an amplitude of a T wave of a PQRST complex, a width of a P wave of a PQRST complex, a width of a QRS wave of a PQRST complex, a width of a T wave of a PQRST complex, a ST segment of a PQRST complex, a coupling interval between a QRS complex and a premature, abnormal complex, an R-R interval between two or more PQRST complexes, a ST deviation, presence of one or more premature ventricular contractions.

11. A defibrillator (1) according to claim 9 or claim 10 in which the refibrillation indicator model is configured to combine the ECG pre-shock refibrillation indicator and the ECG post-shock refibrillation indicator to generate an aggregate ECG refibrillation indicator, to compare the aggregate ECG refibrillation indicator with at least one pre-determined aggregate ECG refibrillation threshold and to generate a flag which prompts the defibrillator to deliver a treatment to the patient.

12. A defibrillator (1) according to claim 11 in which, when the aggregate ECG refibrillation indicator is less than or equal to the at least one pre-determined ECG refibrillation threshold, the refibrillation indicator model is configured to generate a flag which prompts the defibrillator to start CPR treatment of the patient and, when the aggregate ECG refibrillation indicator is greater than the at least one pre-determined ECG refibrillation threshold, the refibrillation indicator model is configured to generate a flag which prompts the defibrillator (1) to delay CPR treatment of the patient.

13. A defibrillator (1) according to any of claims 9 to 12 in which, at one or more further times after delivery of the defibrillation shock, the ECG analysis system (5) is configured to repeat analysis of the patient ECG signal, the refibrillation indicator model is configured to generate a further ECG post-shock refibrillation indicator and to combine the ECG pre-shock refibrillation indicator and the further ECG post-shock refibrillation indicator to generate an aggregate ECG refibrillation indicator, to compare the aggregate ECG refibrillation indicator with at least one pre-determined aggregate ECG refibrillation threshold and to generate a flag which prompts the defibrillator (1) to deliver a treatment to the patient.

14. A defibrillator (1) according to claim 11 in which the refibrillation indicator model is configured to combine the aggregate ECG refibrillation indicator and the at least one impedance refibrillation indicator to generate an aggregate ECG/impedance refibrillation indicator, to compare the aggregate ECG/impedance refibrillation indicator with an aggregate ECG/impedance refibrillation threshold and to generate a flag which prompts the defibrillator (1) to deliver a treatment to the patient.

## Patentansprüche

1. Defibrillator (1) zur Beurteilung der Wahrscheinlichkeit einer Refibrillation eines Patienten, umfassend:
einen EKG-Signaleingang (3), der dazu konfiguriert ist, EKG-Signale des Patienten zu empfangen;
ein EKG-Analysesystem (5) in Kommunikation mit dem EKG-Signaleingang (3), das dazu konfiguriert ist, die Patienten-EKG-Signale zu empfangen, die Patienten-EKG-Signale zu analysieren, um einen EKG-Refibrillationsindikator zu erzeugen, der eine Wahrscheinlichkeit einer Refibrillation des Patienten darstellt, und den EKG-Refibrillationsindikator zu verwenden, um Behandlung für den Patienten zu wählen;
einen Impedanzsignaleingang (7), der dazu konfiguriert ist, Impedanzsignale des Patienten zu empfangen, und
ein Impedanzanalysesystem (9) in Kommunikation mit dem Impedanzsignaleingang (7), das dazu konfiguriert ist, das Patientenimpedanzsignal zu empfangen,
**dadurch gekennzeichnet, dass:**
das Impedanzanalysesystem (9) dazu konfiguriert ist, das Patientenimpedanzsignal zu analysieren, um zumindest einen Impedanzrefibrillationsindikator zu erzeugen, der eine Wahrscheinlichkeit einer Refibrillation des Patienten darstellt, und der Defibrillator dazu konfiguriert ist, auch den zumindest einen Impedanzrefibrillationsindikator zu verwenden, um Behandlung für den Patienten zu wählen.

2. Defibrillator (1) nach Anspruch 1, wobei das EKG-Analysesystem (5) dazu konfiguriert ist, die Patienten-EKG-Signale vor einem Defibrillationsschock zu analysieren und einen EKG-Vorschock-Refibrillationsindikator erzeugt und der Defibrillator (1) den EKG-Vorschock-Refibrillationsindikator verwendet, um Behandlung für den Patienten zu wählen.

3. Defibrillator (1) nach Anspruch 2, der dazu konfiguriert ist, den EKG-Vorschock-Refibrillationsindikator zu verwenden, um zu wählen, dem Patienten Defibrillationsschockbehandlung mit einer Energie gleich einer Standardenergie zu liefern oder dem Patienten Defibrillationsschockbehandlung mit einer Energie größer als die Standardenergie zu liefern.

4. Defibrillator (1) nach Anspruch 2 oder Anspruch 3, wobei das EKG-Analysesystem (5) dazu konfiguriert ist, die Patienten-EKG-Signale nach Lieferung eines Defibrillationsschocks zu analysieren, und zumindest einen EKG-Nachschock-Refibrillationsindikator erzeugt und der Defibrillator (1) dazu konfiguriert ist, den EKG-Vorschock-Refibrillationsindikator und den zumindest einen EKG-Nachschock-Refibrillationsindikator zu kombinieren, um einen aggregierten EKG-Refibrillationsindikator zu erzeugen und den aggregierten EKG-Refibrillationsindikator zu verwenden, um Behandlung für den Patienten zu wählen.

5. Defibrillator (1) nach Anspruch 4, der dazu konfiguriert ist, den aggregierten EKG-Refibrillationsindikator zu verwenden, um zu wählen, CPR-Behandlung des Patienten zu starten oder CPR-Behandlung des Patienten zu verzögern.

6. Defibrillator (1) nach einem vorhergehenden Anspruch, wobei vor Lieferung eines Defibrillationsschocks das EKG-Analysesystem (5) dazu konfiguriert ist, eine oder mehrere Proben der EKG-Signale über einen oder mehrere Zeiträume zu nehmen, eine oder mehrere Proben der EKG-Signale zu analysieren, um einen oder mehrere Parameter der oder jeder Probe zu detektieren und den oder jeden oder zumindest einige der Parameter als einen oder mehrere Eingänge eines Refibrillationsindikatormodells zu verwenden, das dazu konfiguriert ist, einen EKG-Vorschock-Refibrillationsindikator zu erzeugen.

7. Defibrillator (1) nach Anspruch 6, wobei der eine oder die mehreren Parameter beliebige von Zeitdomänenparametern, Frequenzdomänenparametern, Wavelet-Parametern, einer maximalen EKG-Signalprobenamplitude, einer minimalen EKG-Signalprobenamplitude, einer maximalen positiven Steigung einer EKG-Signalprobe, einer minimalen positiven Steigung einer EKG-Signalprobe, einer maximalen negativen Steigung einer EKG-Signalprobe, einer minimalen negativen Steigung einer EKG-Signalprobe, einem maximalen Amplitudenspektrumsbereich (AMSA) einer EKG-Signalprobe, einem minimalen AMSA einer EKG-Signalprobe, einer EKG-Signalprobenschwerpunktfrequenz, einer EKG-Signalprobenleistung, Parametern einer VF-Wellenform sind.

8. Defibrillator (1) nach Anspruch 6 oder Anspruch 7, wobei das Refibrillationsindikatormodell dazu konfiguriert ist, den EKG-Vorschock-Refibrillationsindikator mit zumindest einem vorbestimmten EKG-Vorschock-Refibrillationsschwellenwert zu vergleichen und ein Flag zu erzeugen, das den Defibrillator (1) auffordert, dem Patienten eine Behandlung zu liefern.

9. Defibrillator (1) nach einem vorhergehenden Anspruch, wobei nach Lieferung eines Defibrillationsschocks das EKG-Analysesystem (5) dazu konfiguriert ist, eine oder mehrere Proben der EKG-Signale über einen oder mehrere Zeiträume zu nehmen, eine oder mehrere Proben der EKG-Signale zu analysieren, um einen oder mehrere Parameter der oder jeder Probe zu detektieren und den oder jeden oder zumindest einige der Parameter als einen oder mehrere Eingänge eines Refibrillationsindikatormodells zu verwenden, das einen EKG-Nachschock-Refibrillationsindikator erzeugt.

10. Defibrillator (1) nach Anspruch 9, wobei der eine oder die mehreren Parameter beliebige von Zeitdomänenparametern, Frequenzdomänenparametern, Wavelet-Parametern, einer maximalen EKG-Signalprobenamplitude, einer minimalen EKG-Signalprobenamplitude, einer maximalen positiven Steigung einer EKG-Signalprobe, einer minimalen positiven Steigung einer EKG-Signalprobe, einer maximalen negativen Steigung einer EKG-Signalprobe, einer minimalen negativen Steigung einer EKG-Signalprobe, einem maximalen AMSA einer EKG-Signalprobe, einem minimalen AMSA einer EKG-Signalprobe, einer EKG-Signalprobenschwerpunktfrequenz, einer EKG-Signalprobenleistung, einer Amplitude einer P-Welle eines PQRST-Komplexes, einer Amplitude einer QRS-Welle eines PQRST-Komplexes, Variation von Amplituden von QRS-Wellen von zwei oder mehr PQRST-Komplexen, einer Amplitude einer T-Welle eines PQRST-Komplexes, einer Breite einer P-Welle eines PQRST-Komplexes, einer Breite einer QRS-Welle eines PQRST-Komplexes, einer Breite einer T-Welle eines PQRST-Komplexes, einem ST-Segment eines PQRST-Komplexes, einem Kopplungsintervall zwischen einem QRS-Komplex und einem frühreifen, abnormalen Komplex, einem R-R-Intervall zwischen zwei oder mehr PQRST-Komplexen, einer ST-Abweichung, Vorhandensein von einem oder mehreren frühreifen ventrikulären Kontraktionen sind.

11. Defibrillator (1) nach Anspruch 9 oder Anspruch 10, wobei das Refibrillationsindikatormodell dazu konfiguriert ist, den EKG-Vorschock-Refibrillationsindikator und den EKG-Nachschock-Refibrillationsindikator zu kombinieren, um einen aggregierten EKG-Refibrillationsindikator zu erzeugen, den aggregierten EKG-Refibrillationsindikator mit zumindest einem vorbestimmten aggregierten EKG-Refibrillationsschwellenwert zu vergleichen und ein Flag zu erzeugen, das den Defibrillator auffordert, dem Patienten eine Behandlung zu liefern.

12. Defibrillator (1) nach Anspruch 11, wobei, wenn der aggregierte EKG-Refibrillationsindikator weniger als der oder gleich dem zumindest einen vorbestimmten EKG-Refibrillationsschwellenwert ist, das Refibrillationsindikatormodell dazu konfiguriert ist, ein Flag zu erzeugen, das den Defibrillator auffordert, CPR-Behandlung des Patienten zu starten, und, wenn der aggregierte EKG-Refibrillationsindikator größer als der zumindest eine vorbestimmte EKG-Refibrillationsschwellenwert ist, das Refibrillationsindikatormodell dazu konfiguriert ist, ein Flag zu erzeugen, das den Defibrillator (1) auffordert, CPR-Behandlung des Patienten zu verzögern.

13. Defibrillator (1) nach einem der Ansprüche 9 bis 12, wobei zu einer oder mehreren weiteren Zeiten nach Lieferung des Defibrillationsschocks das EKG-Analysesystem (5) dazu konfiguriert ist, Analyse des Patienten-EKG-Signals zu wiederholen, das Refibrillationsindikatormodell dazu konfiguriert ist, einen weiteren EKG-Nachschock-Refibrillationsindikator zu erzeugen und den EKG-Vorschock-Refibrillationsindikator und den weiteren EKG-Nachschock-Refibrillationsindikator zu kombinieren, um einen aggregierten EKG-Refibrillationsindikator zu erzeugen, den aggregierten EKG-Refibrillationsindikator mit zumindest einem vorbestimmten aggregierten EKG-Refibrillationsschwellenwert zu vergleichen und ein Flag zu erzeugen, das den Defibrillator (1) auffordert, dem Patienten eine Behandlung zu liefern.

14. Defibrillator (1) nach Anspruch 11, wobei das Refibrillationsindikatormodell dazu konfiguriert ist, den aggregierten EKG-Refibrillationsindikator und den zumindest einen Impedanz-Refibrillationsindikator zu kombinieren, um einen aggregierten EKG-/Impedanz-Refibrillationsindikator zu erzeugen, den aggregierten EKG-/Impedanz-Refibrillationsindikator mit einem aggregierten EKG-/Impedanz-Refibrillationsschwellenwert zu vergleichen und ein Flag zu erzeugen, das den Defibrillator (1) auffordert, dem Patienten eine Behandlung zu liefern.

## Revendications

1. Défibrillateur (1) permettant d'évaluer une probabilité de refibrillation de patient, comprenant :
une entrée de signal d'ECG (3) configurée pour recevoir des signaux d'ECG du patient ;
un système d'analyse d'ECG (5), en communication avec l'entrée de signal d'ECG (3), configuré pour recevoir les signaux d'ECG de patient, analyser les signaux d'ECG de patient de manière à générer un indicateur de refibrillation d'ECG représentant une probabilité de refibrillation du patient et utiliser l'indicateur de refibrillation d'ECG de manière à choisir un traitement pour le patient ;
une entrée de signal d'impédance (7) configurée pour recevoir des signaux d'impédance du patient, et
un système d'analyse d'impédance (9), en communication avec l'entrée de signal d'impédance (7), configuré pour recevoir le signal d'impédance du patient,
**caractérisé en ce que :**
le système d'analyse d'impédance (9) est configuré pour analyser le signal d'impédance du patient de manière à générer au moins un indicateur de refibrillation d'impédance représentant une probabilité de refibrillation du patient et le défibrillateur est configuré pour utiliser également ledit au moins un indicateur de refibrillation d'impédance de manière à choisir un traitement pour le patient.

2. Défibrillateur (1) selon la revendication 1, ledit système d'analyse d'ECG (5) étant configuré pour analyser les signaux d'ECG de patient avant un choc de défibrillation et générant un indicateur de refibrillation de pré-choc d'ECG et ledit défibrillateur (1) utilisant l'indicateur de refibrillation de pré-choc d'ECG pour choisir un traitement pour le patient.

3. Défibrillateur (1) selon la revendication 2, qui est configuré pour utiliser l'indicateur de refibrillation de pré-choc d'ECG de manière à choisir d'administrer un traitement de choc de défibrillation au patient à une énergie égale à une énergie par défaut ou d'administrer un traitement de choc de défibrillation au patient à une énergie supérieure à l'énergie par défaut.

4. Défibrillateur (1) selon la revendication 2 ou la revendication 3, ledit système d'analyse d'ECG (5) étant configuré pour analyser les signaux d'ECG de patient après administration d'un choc de défibrillation et générant au moins un indicateur de refibrillation de post-choc d'ECG et ledit défibrillateur (1) étant configuré pour combiner l'indicateur de refibrillation de pré-choc d'ECG et ledit au moins un indicateur de refibrillation de post-choc d'ECG de manière à générer un indicateur de refibrillation d'ECG agrégé et à utiliser l'indicateur de refibrillation d'ECG agrégé afin de choisir un traitement pour le patient.

5. Défibrillateur (1) selon la revendication 4, qui est configuré pour utiliser l'indicateur de refibrillation d'ECG agrégé de manière à choisir de commencer un traitement par RCP du patient ou de retarder un traitement par RCP du patient.

6. Défibrillateur (1) selon l'une quelconque des revendications précédentes, avant administration d'un choc de défibrillation, ledit système d'analyse d'ECG (5) étant configuré pour prendre un ou plusieurs échantillons des signaux d'ECG sur une ou plusieurs périodes de temps, pour analyser un ou plusieurs échantillons des signaux d'ECG de manière à détecter un ou plusieurs paramètres dudit ou de chaque échantillon et à utiliser ledit ou chacun ou au moins certains des paramètres en tant qu'une ou plusieurs entrées d'un modèle d'indicateur de refibrillation qui est configuré pour générer un indicateur de refibrillation de pré-choc d'ECG.

7. Défibrillateur (1) selon la revendication 6, lesdits un ou plusieurs paramètres étant l'un quelconque des paramètres de domaine temporel, des paramètres de domaine fréquentiel, des paramètres d'ondelette, une amplitude maximale d'échantillon de signal d'ECG, une amplitude minimale d'échantillon de signal d'ECG, une pente positive maximale d'échantillon de signal d'ECG, une pente positive minimale d'échantillon de signal d'ECG, une pente négative maximale d'échantillon de signal d'ECG, une pente négative minimale d'échantillon de signal d'ECG, une zone spectrale d'amplitude maximale d'échantillon de signal d'ECG (AMSA), une AMSA minimale d'échantillon de signal d'ECG, une fréquence centroïde d'échantillon de signal d'ECG, une puissance d'échantillon de signal d'ECG, des paramètres d'une forme d'onde VF.

8. Défibrillateur (1) selon la revendication 6 ou la revendication 7, ledit modèle d'indicateur de refibrillation étant conçu pour comparer l'indicateur de refibrillation de pré-choc d'ECG à au moins un seuil de refibrillation de pré-choc d'ECG prédéfini et pour générer un indicateur qui invite le défibrillateur (1) à administrer un traitement au patient.

9. Défibrillateur (1) selon l'une quelconque des revendications précédentes, après administration d'un choc de défibrillation, ledit système d'analyse d'ECG (5) étant configuré pour prendre un ou plusieurs échantillons des signaux d'ECG sur une ou plusieurs périodes de temps, pour analyser un ou plusieurs échantillons des signaux d'ECG de manière à détecter un ou plusieurs paramètres dudit ou de chaque échantillon et pour utiliser ledit ou chacun ou au moins certains des paramètres en tant qu'une ou plusieurs entrées d'un modèle d'indicateur de refibrillation qui génère un indicateur de refibrillation de post-choc d'ECG.

10. Défibrillateur (1) selon la revendication 9, lesdits un ou plusieurs paramètres étant l'un quelconque des paramètres de domaine temporel, des paramètres de domaine fréquentiel, des paramètres d'ondelette, une amplitude maximale d'échantillon de signal d'ECG, une amplitude minimale d'échantillon de signal d'ECG, une pente positive maximale d'échantillon de signal d'ECG, une pente positive minimale d'échantillon de signal d'ECG, une pente négative maximale d'échantillon de signal d'ECG, une pente négative minimale d'échantillon de signal d'ECG, une AMSA maximale d'échantillon de signal d'ECG, une AMSA minimale d'échantillon de signal d'ECG, une fréquence centroïde d'échantillon de signal d'ECG, une puissance d'échantillon de signal d'ECG, une amplitude d'une onde P d'un complexe PQRST, une amplitude d'une onde QRS d'un complexe PQRST, une variation d'amplitudes d'ondes QRS de deux complexes PQRST ou plus, une amplitude d'une onde T d'un complexe PQRST, une largeur d'une onde P d'un complexe PQRST, une largeur d'une onde QRS d'un complexe PQRST, une largeur d'une onde T d'un complexe PQRST, un segment ST d'un complexe PQRST, un intervalle de couplage entre un complexe QRS et un complexe prématuré anormal, un intervalle R-R entre deux complexes PQRST ou plus, une déviation ST, la présence d'une ou plusieurs contractions ventriculaires prématurées.

11. Défibrillateur (1) selon la revendication 9 ou la revendication 10, ledit modèle d'indicateur de refibrillation étant configuré pour combiner l'indicateur de refibrillation de pré-choc d'ECG et l'indicateur de refibrillation de post-choc d'ECG de manière à générer un indicateur de refibrillation d'ECG agrégé, pour comparer l'indicateur de refibrillation d'ECG agrégé à au moins un seuil de refibrillation d'ECG agrégé prédéfini et pour générer un indicateur qui invite le défibrillateur à administrer un traitement au patient.

12. Défibrillateur (1) selon la revendication 11, lorsque l'indicateur de refibrillation d'ECG agrégé est inférieur ou égal audit au moins un seuil de refibrillation d'ECG prédéfini, ledit modèle d'indicateur de refibrillation étant configuré pour générer un indicateur qui invite le défibrillateur à commencer un traitement par RCP du patient et, lorsque l'indicateur de refibrillation d'ECG agrégé est supérieur audit au moins un seuil de refibrillation d'ECG prédéfini, ledit modèle d'indicateur de refibrillation étant configuré pour générer un indicateur qui invite le défibrillateur (1) à retarder le traitement par RCP du patient.

13. Défibrillateur (1) selon l'une quelconque des revendications 9 à 12, ledit système d'analyse d'ECG (5) étant configuré pour répéter l'analyse du signal d'ECG de patient, ledit modèle d'indicateur de refibrillation étant configuré pour générer un autre indicateur de refibrillation de post-choc d'ECG et pour combiner l'indicateur de refibrillation de pré-choc d'ECG et l'autre indicateur de refibrillation de post-choc d'ECG afin de générer un indicateur de refibrillation d'ECG agrégé, pour comparer l'indicateur de refibrillation d'ECG agrégé à au moins un seuil de refibrillation d'ECG agrégé prédéfini et pour générer un indicateur qui invite le défibrillateur (1) à administrer un traitement au patient.

14. Défibrillateur (1) selon la revendication 11, ledit modèle d'indicateur de refibrillation étant configuré pour combiner l'indicateur de refibrillation d'ECG agrégé et ledit au moins un indicateur de refibrillation d'impédance de manière à générer un indicateur de refibrillation d'ECG/impédance agrégé, pour comparer l'indicateur de refibrillation d'ECG/impédance agrégé à un seuil de refibrillation d'ECG/impédance agrégé et pour générer un indicateur qui invite le défibrillateur (1) à administrer un traitement au patient.
